# FASCICULE DE BREVET EUROPEEN

(11) **EP 1 229 844 B1**
(45) Date de publication et mention de la délivrance du brevet: **26.10.2005**
(21) Numéro de dépôt: 00983372.4
(22) Date de dépôt: 15.11.2000
(51) Int. Cl.: A61B 17/16, A61B 17/32

(54) **PINCE-GOUGE A UTILISATION MEDICALE**
RILLENZANGE ZUR MEDIZINISCHEN VERWENDUNG
GOUGE FORCEPS FOR MEDICAL USE

(30) Priorité: 17.11.1999 FR 9914464; 19.07.2000 FR 0009501
(43) Date de publication de la demande: 14.08.2002
(73) Titulaire: Favreul, Emmanuel, 69110 Sainte Foy Les Lyon (FR)
(72) Inventeur: Favreul, Emmanuel, 69110 Sainte Foy Les Lyon (FR)
(74) Mandataire: Maureau, Philippe
(86) Numéro de dépôt international: PCT/FR2000/003177
(87) Numéro de publication internationale: WO 2001/035838

(56) Documents cités:
- DE-U- 29 718 969
- US-A- 4 586 497
- US-A- 5 451 227
- US-A- 5 484 441

## Description

La présente invention concerne une pince-gouge à utilisation médicale, permettant la taille d'un os par des coupes répétées de fragments de cet os.

Une telle pince, couramment dénommée "pince de Kerrison" ou "rongeur", est utilisée pour tailler un os en vue de la mise en place d'un implant ou de la libération d'un canal anatomique, au niveau de sites anatomiques délicats ou difficiles d'accès. Ce genre de pince est en particulier utilisé dans le domaine de la chirurgie du rachis.

De manière classique, une pince-gouge présente une embase et un coulisseau allongés, le coulisseau étant déplaçable sur cette embase. L'embase est pourvue, à son extrémité distale, d'un bec recourbé formant une enclume, et le coulisseau présente une chambre débouchant au niveau de son extrémité distale par une ouverture délimitée par un bord tranchant.

Une telle pince est décrite dans le document US-A-5484441.

Pour la coupe d'un fragment osseux, l'enclume est placée au-delà de la partie osseuse à tailler et l'extrémité distale du coulisseau est placée en deçà de cette même partie osseuse, puis le coulisseau est déplacé par rapport à l'embase. Le bord tranchant distal du coulisseau sectionne un fragment d'os, qui s'engage dans ladite chambre au fur et à mesure de sa coupe.

La pince est ensuite retirée pour extraire le fragment de la chambre puis est réintroduite en vue de la coupe d'un autre fragment osseux.

Dans certains cas, de multiples coupes doivent être opérées, de sorte que les opérations d'extraction puis d'introduction de la pince sont répétées de nombreuses fois. La taille d'un os peut dès lors être longue et fastidieuse à réaliser.

De plus, les pinces existantes ont une certaine tendance au coincement de fragments osseux dans ladite chambre, obligeant à un démontage du coulisseau pour extraire le fragment. Ces démontages occasionnent du travail et des pertes de temps supplémentaires.

Parfois, les fragments ne sont pas parfaitement captés par ladite chambre et peuvent être projetés, compte tenu de la force exercée. Ces fragments doivent alors être retrouvés et retirés, ce qui occasionne également des pertes de temps.

En outre, les fragments osseux ont une certaine tendance à s'insérer dans les moyens distaux de guidage du coulisseau par rapport à l'embase, qui se trouvent à proximité de la zone de coupe. L'insertion d'un fragment conduit au coincement du coulisseau sur l'embase, ou vient empêcher la venue de l'extrémité distale du coulisseau au contact de l'enclume, ce qui empêche la coupe complète d'un fragment.

La présente invention vise à remédier à l'ensemble de ces inconvénients.

La pince qu'elle concerne comprend, de manière connue en soi, une embase et un coulisseau allongés, le coulisseau étant déplaçable sur cette embase laquelle est pourvue, à son extrémité distale, d'un bec recourbé formant une enclume, et le coulisseau présentant une chambre débouchant au niveau de son extrémité distale par une ouverture délimitée par un bord tranchant. Selon l'invention, la face de l'embase le long de laquelle se déplace le coulisseau est lisse au niveau distal, et l'embase présente un arceau à l'intérieur duquel le coulisseau est engagé et peut coulisser, en étant guidé par cet arceau, sans aucun moyen de guidage de type rainure/nervure aux abords de la chambre.

La pince selon l'invention ne comprend ainsi, à la différence des pinces existantes, aucun moyen de guidage de type rainure/nervure aux abords de la chambre de réception du fragment sectionné. Le risque d'insertion de ce fragment dans une telle rainure ou entre la rainure et la nervure est ainsi éliminé.

Ledit arceau permet en outre de parfaitement contenir les parois latérales du coulisseau au niveau de ladite chambre. Il en résulte que toute déformation latérale du coulisseau, pouvant se produire sous l'effet des contraintes générées par la coupe d'un fragment, est éliminée.

Dès lors, et selon une caractéristique avantageuse de l'invention, ladite chambre débouche dans la face du coulisseau tournée vers l'embase, de sorte que le coulisseau présente une section transversale en forme de U inversé au niveau de cette chambre.

Cette absence de fond au niveau de la chambre réduit encore le risque de coincement d'un fragment d'os dans cette chambre.

Selon un autre aspect de l'invention, le biseau permettant de constituer le bord tranchant distal du coulisseau est aménagé sur l'extérieur du coulisseau.

Ce biseau n'est donc pas aménagé sur l'intérieur du coulisseau comme sur les pinces de la technique antérieure, et permet de découper un fragment ayant la même section transversale que celle de la chambre.

Les risques de coincement du fragment ou de mauvais captage de ce fragment s'en trouvent fortement réduits.

Selon un autre aspect de l'invention, ledit bec présente un bossage proximal autour duquel le coulisseau vient en engagement en fin de course de coupe d'un fragment.

Ce bossage permet d'assurer la parfaite introduction d'un fragment d'os dans la chambre.

Ledit bossage peut avoir une forme et des dimensions qui correspondent, en section transversale, à celles de la chambre. La complète section du fragment est ainsi assurée. Ce bossage permet également d'assurer un positionnement stable de la pince-gouge avant le déplacement du coulisseau.

Selon encore un autre aspect de l'invention, la pince présente une chambre propre à recevoir une pluralité de fragments.

Il n'est dès lors pas nécessaire de retirer la pince après chaque coupe d'un fragment, les différents fragments s'engageant dans la chambre les uns à la suite des autres.

Avantageusement, cette chambre présente une section qui augmente dans la direction proximale.

Le volume de cette chambre est ainsi progressivement croissant du côté distal vers le côté proximal, de telle sorte que la chambre a une capacité importante et peut recevoir les fragments accumulés lors des diverses coupes successives. De plus et surtout, les frottements de ces fragments contre les parois du coulisseau qui délimitent cette chambre se réduisent au fur et à mesure de l'avancement d'un fragment dans cette chambre, ce qui réduit et même élimine tout risque de coincement.

De préférence, l'embase comprend une creusure au droit de la chambre, qui permet d'augmenter encore le volume de cette chambre.

Cette creusure peut avoir une largeur et une profondeur constantes au long de la chambre mais présente avantageusement une largeur et/ou une profondeur qui augmente(nt) dans la direction proximale de la pince-gouge, pour augmenter encore le volume de la chambre et suivre l'augmentation précitée de la section de cette chambre.

Cette creusure peut notamment présenter une profondeur qui augmente progressivement dans la direction proximale, ce qui contribue à favoriser le glissement des fragments dans ladite chambre et ladite creusure lors de la progression de ces fragments dans la direction distale.

Pour sa bonne compréhension, l'invention est à nouveau décrite ci-dessous en référence au dessin schématique annexé représentant, à titre d'exemple non limitatif, une forme de réalisation préférée de la pince-gouge qu'elle concerne.
La figure 1 en est une vue en perspective éclatée ;
la figure 2 en est une vue de côté, en coupe longitudinale ;
la figure 3 en est une vue en coupe selon la ligne III-III de la figure 2 ;
les figures 4 à 6 en sont des vues en coupe selon respectivement les lignes IV-IV, V-V et VI-VI de la figure 2 et
les figures 7 et 8 en sont des vues en coupe longitudinale, selon une variante, au niveau de son extrémité distale, respectivement en position reculée et avancée du coulisseau qu'elle comprend.

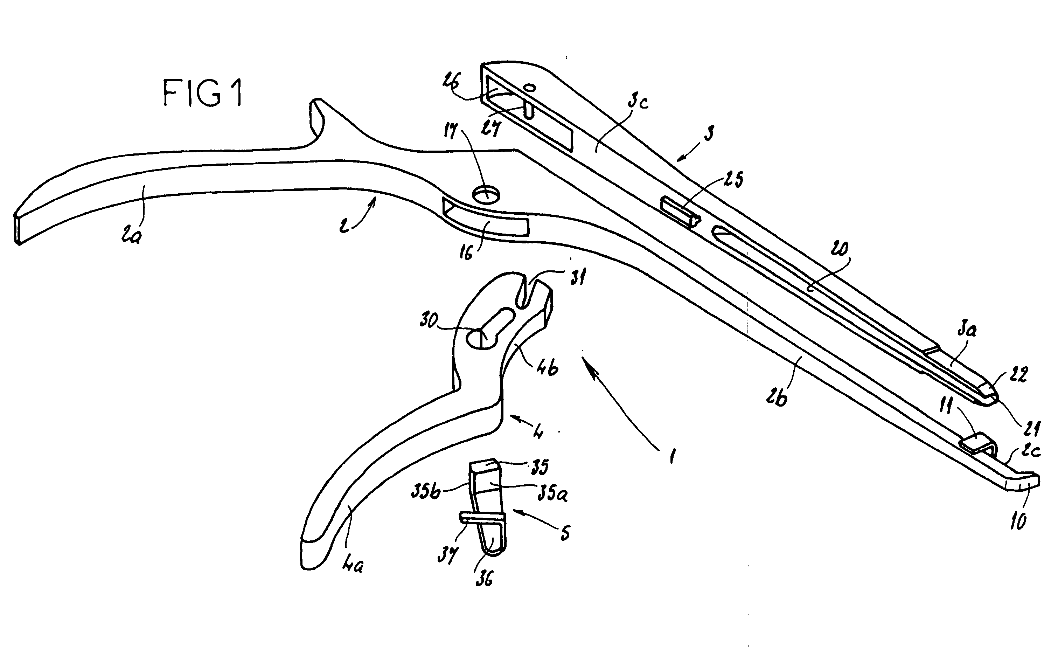

Les figures 1 à 6 représentent une pince-gouge 1 à utilisation médicale, permettant la taille d'un os par des coupes répétées de fragments de cet os.

Dans l'exemple représenté, cette pince-gouge 1 comprend une embase 2, un coulisseau 3, une pièce 4 en forme de gâchette et une pièce 5 formant l'axe de pivotement de la pièce 4.

L'embase 2 présente une partie 2a en forme de crosse, destinée à prendre appui contre la paume d'une main, et une partie allongée 2b présentant une face plane 2c, le long de laquelle se déplace le coulisseau 3.

Cette embase 2 comprend à son extrémité distale, c'est-à-dire opposée à la crosse 2a, un bec 10 et un arceau 11. Le bec 10 fait saillie du côté de la partie 2b opposé à celui sur lequel se trouve la crosse 2a et est pourvu d'un bossage 12 faisant saillie de sa face proximale. L'arceau 11 présente une forme en U inversé et est fixé, par ses branches latérales, aux faces latérales de la partie 2b.

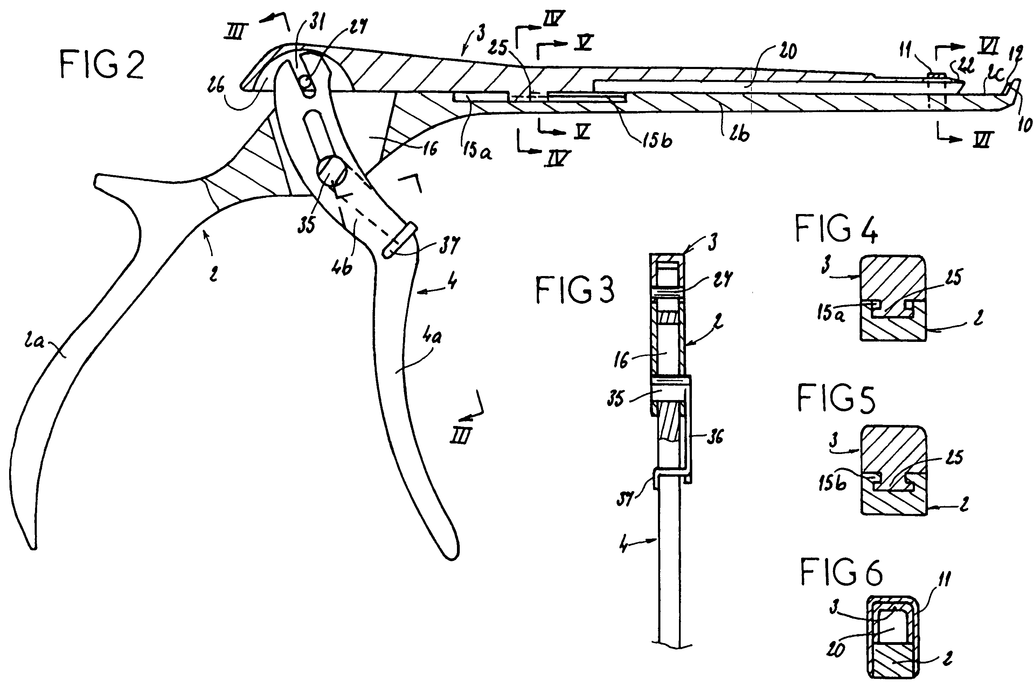

La face 2c est lisse du côté distal, sur une large moitié de sa longueur. Du côté proximal, l'embase 2 présente une rainure longitudinale débouchant dans cette face 2c. Cette rainure présente une partie proximale 15a, montrée à la figure 4, dans laquelle elle a une forme purement rectangulaire et une partie distale 15b dans laquelle elle a un profil en T inversé.

L'embase 2 comprend en outre une lumière 16 s'étendant dans le même plan que la crosse 2a, destinée à recevoir la gâchette 4, et deux trous 17 coaxiaux, perpendiculaires à cette lumière 16.

Le coulisseau 3 présente une chambre 20 aménagée sur une large moitié de sa longueur, au niveau distal. Cette chambre 20 débouche à l'extrémité distale de ce coulisseau 3 par une ouverture 21 ainsi que dans ladite face 3c.

Cette chambre 20 a une section transversale qui va en s'élargissant dans le sens proximal.

L'ouverture 21 est délimitée par un bord tranchant 22. La forme tranchante de ce bord résulte d'un biseau aménagé dans le coulisseau 3 à partir des faces extérieures de celui-ci.

La forme du coulisseau 3 au niveau de l'ouverture 21 correspond à la forme du bossage 12, de telle sorte que le coulisseau 3 s'engage sur ce bossage 12 lorsque son extrémité distale vient au contact du bec 10.

Le coulisseau 3 présente en outre une partie d'extrémité distale 3a dont la forme extérieure correspond à la forme intérieure de l'arceau 11. Cette partie 3a peut ainsi être engagée et coulisser sans jeu latéral au travers dudit arceau 11.

Du côté proximal, le coulisseau 3 présente une nervure 25 à profil en forme de T inversé. Cette nervure 25 est propre à être engagée dans la partie de rainure 15a, pour permettre le montage du coulisseau 3 sur l'embase 2, puis à être engagée et à coulisser dans la partie de rainure 15b, en étant retenue par celle-ci.

A son extrémité proximale, le coulisseau 3 présente un évidement 26 de forme hémi-circulaire, ainsi qu'un tourillon 27 d'axe transversal au coulisseau 3.

La pièce 4 en forme de gâchette présente quant à elle une partie courbe 4a sur laquelle les doigts d'une main viennent prendre appui lorsque la partie 2a est placée dans la paume de cette même main, et une partie 4b engagée au travers de la lumière 16 puis dans l'évidement 26.

Il se déduit de la figure 2 que la gâchette 4 constitue un levier permettant de déplacer le coulisseau 3 selon un mouvement de va-et-vient par rapport à l'embase 2.

Ainsi que cela apparaît plus particulièrement sur les figures 1 et 2, la partie 4b comprend une lumière longitudinale 30 présentant une partie allongée et une partie circulaire de même diamètre que les trous 17. Cette même partie 4b comprend également une encoche supérieure 31 destinée à être engagée et à pivoter autour du tourillon 27.

La pièce 5 comprend une broche aplatie 35, un bras 36 et un cavalier 37.

La broche 35 présente deux méplats latéraux 35a et deux faces d'extrémité 35b inscrites dans un cercle de même diamètre que les trous 17 et partie circulaire de la lumière 30. Cette broche 35 peut ainsi, comme montré à la figure 2, être engagée dans les trous 17 et au travers de la partie circulaire de la lumière 30. Lorsque les méplats 35a sont parallèles à la partie allongée de la lumière 30, la gâchette 4 peut coulisser par rapport à cette broche 35 de manière à permettre le retrait de la gâchette 4 d'autour du tourillon 27 et donc le démontage du coulisseau 3 ; lorsque ces méplats 35a sont non parallèles à la partie allongée de la lumière 30, position représentée à la figure 2, la broche 35 ne permet que le pivotement de la gâchette 4.

Le bras 36 et le cavalier 37 sont tels que ce cavalier 37 peut être engagé avec frottements autour de la gâchette 4 lorsque la broche 35 est engagée dans les trous 17 et la partie circulaire de la lumière 30.

Il se déduit de la figure 2 que, lorsque le cavalier 37 est engagé autour de la gâchette 4, la broche 35 n'est pas parallèle à la partie rectiligne de la lumière 30 et assure ainsi le montage et le pivotement de cette gâchette 4. Le cavalier 37 peut être dégagé de la partie 4b et la pièce 5 peut être pivotée jusqu'à amener les méplats 35a parallèlement à la partie rectiligne de la lumière 30, ce qui permet le coulissement de la gâchette 4 comme indiqué plus haut.

En pratique, pour la coupe d'un fragment osseux, le bec 10 est placé au-delà de la partie osseuse à tailler et l'extrémité distale du coulisseau 3 est placée en deçà de cette même partie osseuse, puis le coulisseau 3 est déplacé par rapport à l'embase 2 au moyen de la gâchette 4. Le bord tranchant 22 sectionne un fragment d'os de même section transversale que la chambre 20 à son extrémité distale.

Ce fragment s'engage dans la chambre 20 au fur et à mesure de sa coupe, et, en fin de course de coupe, le bossage 12 permet d'assurer une coupe nette et une parfaite introduction de ce fragment d'os dans la chambre 20.

Ces opérations peuvent être répétées sans dégager la pince 1 du site à traiter, une série de fragments pouvant s'accumuler dans la chambre 20. Cette dernière a une capacité importante, de par sa section qui augmente dans la direction proximale, et permet que les frottements des fragments contre les parois du coulisseau 3 se réduisent au fur et à mesure de l'avancement d'un fragment.

Lorsque la chambre 20 est pleine, la pince 1 est retirée puis démontée de la manière précitée afin de vider cette chambre 20.

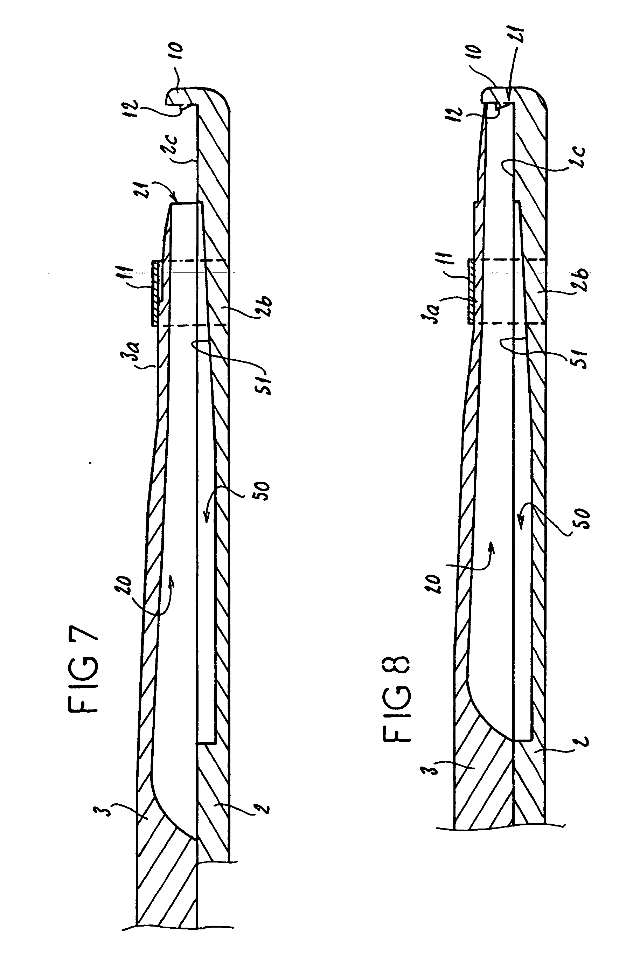

Les figures 7 et 8 représentent une variante de réalisation de la pince-gouge 1. Les éléments déjà décrits qui se retrouvent dans cette variante sont désignés par les mêmes références numériques, même si ces éléments présentent une forme légèrement différente de celle représentée sur les figures précédentes, en particulier en ce qui concerne le bec 10.

Dans cette variante, l'embase 2 comprend une creusure 50 au droit de la chambre 20.

Cette creusure s'étend entre le point à l'aplomb duquel se trouve l'extrémité distale du coulisseau 3 dans la position reculée de ce coulisseau (cf. figure 7) et le point à l'aplomb duquel se trouve la paroi du coulisseau 3 qui délimite l'extrémité proximale de la chambre 20, dans la position avancée du coulisseau 3 (cf. figure 8).

La paroi 2c est donc lisse autour de cette creusure 50, c'est-à-dire au niveau de l'extrémité distale de l'embase 2 et au droit des parois latérales du coulisseau 3 qui délimitent la chambre 20.

La creusure 50 présente un fond 51 dont le tiers distal est incliné, de telle sorte que la profondeur de cette creusure 50 augmente progressivement dans la direction proximale.

La creusure 50 permet d'augmenter encore le volume de stockage des fragments osseux, et la partie inclinée de son fond 51 contribue à favoriser le glissement de ces fragments lors de leur progression dans la direction proximale.

Il apparaît ainsi que l'invention fournit une pince-gouge à utilisation médicale qui remédie aux différents inconvénients des pince-gouges homologues de la technique antérieure. En effet, cette pince 1 ne comprend aucun moyen de guidage de type rainure/nervure aux abords de la chambre 20 ; au contraire, la face 2c est lisse et la chambre 20 débouche dans la face 3c du coulisseau 3, ce qui élimine le risque de coincement d'un fragment. L'arceau 11 permet de parfaitement contenir les parois latérales du coulisseau 3 au niveau de la chambre 20 afin que toute déformation latérale de ce coulisseau 3, pouvant se produire sous l'effet des contraintes générées par la coupe d'un fragment, soit éliminée.

## Revendications

1. Pince-gouge (1) à utilisation médicale, comprenant une embase (2) et un coulisseau (3) allongés, le coulisseau (3) étant déplaçable sur cette embase (2), laquelle est pourvue, à son extrémité distale, d'un bec recourbé (10) formant une enclume, le coulisseau (3) présentant une chambre (20) débouchant au niveau de son extrémité distale par une ouverture (21) délimitée par un bord tranchant (22) et
la face (2c) de l'embase (2) le long de laquelle se déplace le coulisseau (3) étant lisse au niveau distal; pince (1) **caractérisée en ce que** l'embase (2) présente un arceau (11) à l'intérieur duquel le coulisseau (3) est engagé et peut coulisser en étant guidé par cet arceau (11), sans aucun moyen de guidage de type rainure/nervure aux abords de la chambre (20).

2. Pince-gouge (1) à utilisation médicale selon la revendication 1, **caractérisée en ce que** ladite chambre (20) débouche dans la face (3c) du coulisseau (3) tournée vers l'embase (2), de sorte que le coulisseau (3) présente une section transversale en forme de U inversé au niveau de cette chambre (20).

3. Pince-gouge (1) selon la revendication 1 ou la revendication 2, **caractérisée en ce que** le biseau permettant de constituer le bord tranchant distal (22) du coulisseau (3) est aménagé sur l'extérieur du coulisseau (3).

4. Pince-gouge (1) selon l'une des revendications 1 à 3, **caractérisée en ce que** ledit bec (10) présente un bossage proximal (12) autour duquel le coulisseau (3) vient en engagement en fin de course de coupe d'un fragment.

5. Pince-gouge (1) selon la revendication 4, **caractérisée en ce que** le bossage a une forme et des dimensions qui correspondent, en section transversale, à celles de la chambre (20).

6. Pince-gouge (1) selon l'une des revendications 1 à 5, **caractérisée en ce qu'**elle présente une chambre (20) propre à recevoir une pluralité de fragments.

7. Pince-gouge (1) selon la revendication 6, **caractérisée en ce que** la chambre (20) présente une section qui augmente dans la direction proximale.

8. Pince-gouge (1) selon l'une des revendications 1 à 7, **caractérisée en ce que** le coulisseau (3) présente une partie d'extrémité distale (3a) dont la forme extérieure correspond à la forme intérieure de l'arceau (11), de telle sorte que cette partie (3a) peut être engagée et coulisser sans jeu latéral au travers dudit arceau (11).

9. Pince-gouge (1) selon l'une des revendications 1 à 8, **caractérisée en ce que**, du côté proximal, le coulisseau (3) présente une nervure (25) à profil en forme de T inversé tandis que l'embase (2) présente une rainure longitudinale débouchant dans sa face (2c), cette rainure présentant une partie proximale (15a) dans laquelle elle a une forme purement rectangulaire et une partie distale (15b) dans laquelle elle a un profil en T inversé ; la nervure (25) est propre à être engagée dans la partie de rainure (15a), pour permettre le montage du coulisseau (3) sur l'embase (2), puis à être engagée et à coulisser dans la partie de rainure (15b), en étant retenue par celle-ci.

10. Pince-gouge (1) selon l'une des revendications 1 à 9, **caractérisée en ce que** l'embase (2) comprend une partie (2a) en forme de crosse, destinée à prendre appui contre la paume de la main qui saisit la pince (1), et une pièce (4) en forme de gâchette, présentant une partie courbe (4a) sur laquelle les doigts d'une main viennent prendre appui lorsque ladite partie (2a) est placée dans la paume de cette même main.

11. Pince-gouge (1) selon l'une des revendications 1 à 10, **caractérisée en ce que** l'embase (2) comprend une creusure (50) au droit de la chambre (20).

12. Pince-gouge (1) selon la revendication 11, **caractérisée en ce que** la creusure (50) a une largeur et/ou une profondeur qui augmente(nt) dans la direction proximale de la pince-gouge (1).

13. Pince-gouge (1) selon la revendication 12, **caractérisée en ce que** la creusure (50) présente une profondeur qui augmente progressivement dans la direction proximale.

## Patentansprüche

1. Hohlmeissel-Zange (1) zur medizinischen Verwendung, welche eine Basis (2) und einen länglichen Schlitten (3) aufweist, wobei der Schlitten (3) auf der Basis (2) verschiebbar ist, die an ihrem distalen Ende mit einem gekrümmten Schnabel (10) versehen ist, der einen Amboss bildet, wobei der Schlitten (3) eine Kammer (20) aufweist, die an ihrem distalen Ende an einer durch eine Schneidkante (22) begrenzten Öffnung (21) mündet, und wobei die Fläche (2c) der Basis (2), entlang der sich der Schlitten (3) bewegt, an ihrem distalen Ende glatt ist, wobei die Zange (1) **dadurch gekennzeichnet ist, dass** die Basis (2) einen Bügel (11) besitzt, innerhalb dessen der Schlitten (3) eingefügt ist und gleiten kann, wobei er durch den Bügel (11) geführt wird, wobei keinerlei Führungsmittel vom Rille/Rippe-Typ am Eingang der Kammer (20) vorgesehen ist.

2. Hohlmeissel-Zange (1) zur medizinischen Verwendung nach Anspruch 1, **dadurch gekennzeichnet, dass** die Kammer (20) an der der Basis (2) zuwandten Fläche (3c) des Schlittens (3) derart mündet, dass der Schlitten (3) einen Querschnitt in Form eines umgekehrten U bei dieser Kammer (20) hat.

3. Hohlmeissel-Zange (1) nach Anspruch 1 oder 2, **dadurch gekennzeichnet, dass** die Abschrägung, welche die Bildung der distalen Schneidkante (22) des Schlittens (3) ermöglicht, an der Außenseite des Schlittens (3) angeordnet ist.

4. Hohlmeissel-Zange (1) nach einem der Ansprüche 1 bis 3, **dadurch gekennzeichnet, dass** der Schnabel (10) einen proximalen Höcker (12) besitzt, um den herum der Schlitten (3) am Hubende eines Fragmentschnittes in Eingriff gelangt.

5. Hohlmeissel-Zange (1) nach Anspruch 4, **dadurch gekennzeichnet, dass** der Höcker eine Form und Abmessungen hat, welche im Querschnitt denjenigen der Kammer (20) entsprechen.

6. Hohlmeissel-Zange (1) nach einem der Ansprüche 1 bis 5, **dadurch gekennzeichnet, dass** sie eine Kammer (20) besitzt, die eine Vielzahl von Fragmenten aufnehmen kann.

7. Hohlmeissel-Zange (1) nach Anspruch 6, **dadurch gekennzeichnet, dass** die Kammer (20) einen Querschnitt hat, der in der proximalen Richtung zunimmt.

8. Hohlmeissel-Zange (1) nach einem der Ansprüche 1 bis 7, **dadurch gekennzeichnet, dass** der Schieber (3) einen distalen Endabschnitt (3a) besitzt, dessen äußere Form der inneren Form des Bügels (11) entspricht, so dass dieser Abschnitt (3a) ohne seitliches Spiel durch den Bügel (11) eingeführt und in ihm gleiten kann.

9. Hohlmeissel-Zange (1) nach einem der Ansprüche 1 bis 8, **dadurch gekennzeichnet, dass** auf der proximalen Seite der Schlitten (3) eine Profilrippe (25) in Form eines umgekehrten T besitzt, während die Basis (2) eine an ihrer Fläche (2c) mündende Längsrille besitzt, wobei diese Rille einen proximalen Abschnitt (15a) besitzt, in welchem sie rein rechteckförmig ist, und einen distalen Abschnitt (15b) besitzt, in welchem sie ein Profil in Form eines umgekehrten T hat, wobei die Rippe (25) in den Rillenabschnitt (15a) eingeführt werden kann, um die Montage des Schlittens (3) auf der Basis (2) zu ermöglichen, und anschließend in den Rillenabschnitt (15b) eingeführt werden kann und in ihm gleiten kann, wobei sie durch diese gehalten wird.

10. Hohlmeissel-Zange (1) nach einem der Ansprüche 1 bis 9, **dadurch gekennzeichnet, dass** die Basis (2) einen griffförmigen Abschnitt (2a), der dazu bestimmt ist, um an der Handfläche einer die Zange (1) ergreifenden Hand anzuliegen, sowie ein auslöserförmiges Teil (4) aufweist, das einen gekrümmten Abschnitt (4a) besitzt, auf dem die Finger einer Hand anliegen, wenn der Abschnitt (2a) in die Handfläche dieser Hand gelegt wird.

11. Hohlmeissel-Zange (1) nach einem der Ansprüche 1 bis 10, **dadurch gekennzeichnet, dass** die Basis (2) eine Einsenkung (50) gegenüber von der Kammer (20) aufweist.

12. Hohlmeissel-Zange (1) nach Anspruch 11, **dadurch gekennzeichnet, dass** die Einsenkung (50) eine Breite und/oder eine Tiefe hat, die in der proximalen Richtung der Hohlmeissel-Zange (1) zunimmt/zunehmen.

13. Hohlmeissel-Zange (1) nach Anspruch 12, **dadurch gekennzeichnet, dass** die Einsenkung (50) eine Tiefe hat, die in der proximalen Richtung nach und nach zunimmt.

## Claims

1. Bone-cutting forceps (1) for medical use, comprising an elongate slide (3) and base (2), the slide (3) being able to be displaced on this base (2), which is provided at its distal end with a curved nose (10) which forms an anvil, the slide (3) having a chamber (20) which opens at its distal end in an opening (21) which is delimited by a cutting edge (22), and the surface (2c) of the base (2) along which the slide (3) is displaced being smooth at the distal level, forceps (1) **characterised in that** the base (2) has an arch (11) inside which the slide (3) is engaged, and can slide whilst being guided by this arch (11) without any guiding means of the groove/rib type at the approaches to the chamber (20).

2. Bone-cutting forceps (1) for medical use according to claim 1, **characterised in that** the said chamber (20) opens into the surface (3c) of the slide (3) which faces the base (2), such that the slide (3) has a transverse cross-section in the shape of an inverted "U" at this chamber (20).

3. Bone-cutting forceps (1) according to claim 1 or claim 2, **characterised in that** the bevel which makes it possible to constitute the distal cutting edge (22) of the slide (3) is provided on the exterior of the slide (3).

4. Bone-cutting forceps (1) according to any one of claims 1 to 3, **characterised in that** the said nose (10) has a proximal boss (12) around which the slide (3) is engaged at the end of the course of cutting of a fragment.

5. Bone-cutting forceps (1) according to claim 4, **characterised in that** the boss has a shape and dimensions which correspond in transverse cross-section to those of the chamber (20).

6. Bone-cutting forceps (1) according to any one of claims 1 to 5, **characterised in that** it has a chamber (20) which can receive a plurality of fragments.

7. Bone-cutting forceps (1) according to claim 6, **characterised in that** the chamber (20) has a cross-section which increases in the proximal direction.

8. Bone-cutting forceps (1) according to any one of claims 1 to 7, **characterised in that** the slide (3) has a distal end part (3a), the outer shape of which corresponds to the inner shape of the arch (11), such that this part (3a) can be engaged and slide without lateral play through the said arch (11).

9. Bone-cutting forceps (1) according to any one of claims 1 to 8, **characterised in that**, on the proximal side, the slide (3) has a rib (25) with a profile in the shape of an inverted "T", whereas the base (2) has a longitudinal groove which opens into its surface (2c), this groove having a proximal part (15a) in which it has a purely rectangular shape, and a distal part (15b) in which it has a profile in the shape of an inverted "T"; the rib (25) can be engaged in the groove part (15a) in order to permit fitting of the slide (3) on the base (2), and can then be engaged and slide in the groove part (15b) whilst being retained by the latter.

10. Bone-cutting forceps (1) according to any one of claims 1 to 9, **characterised in that** the base (2) comprises a part (2a) in the shape of a cross which is designed to be supported against the palm of the hand of the person who is holding the forceps (1), and a part (4) in the shape of a trigger, which has a curved part (4a) on which the fingers of one hand are supported when the said part (2a) is placed in the palm of this same hand.

11. Bone-cutting forceps (1) according to any one of claims 1 to 10, **characterised in that** the base (2) comprises a hollow (50) at right-angles to the chamber (20).

12. Bone-cutting forceps (1) according to claim 11, **characterised in that** the hollow (50) has a width and/or depth which increase in the proximal direction of the bone-cutting forceps (1).

13. Bone-cutting forceps (1) according to claim 12, **characterised in that** the hollow (50) has a depth which increases progressively in the proximal direction.
